# EUROPEAN PATENT APPLICATION

(11) **EP 3 903 779 A1**
(43) Date of publication of application: **03.11.2021**
(21) Application number: 19904233.4
(22) Date of filing: 26.12.2019
(51) Int. Cl.: A61K 31/40, A61P 3/10, A61P 31/12, A61P 35/00, A01H 1/06, C12P 19/44, A61K 36/28, C12N 5/04, A23L 5/00, A23L 33/105, A01H 5/00, A01H 5/02, A01H 5/06, A01H 5/10, A01H 5/12

(54) **BROUSSONETINE-RICH STEVIA PLANT**

(30) Priority: 28.12.2018 JP 2018248697
(71) Applicant: Suntory Holdings Limited, Osaka 530-8203 (JP)
(72) Inventor: HIRAI Tadayoshi, Soraku-gun, Kyoto 619-0284 (JP); IWAKI Kazunari, Kawasaki-shi, Kanagawa 211-0067 (JP); MIYAGAWA Katsuro, Soraku-gun, Kyoto 619-0284 (JP)
(74) Representative: Mewburn Ellis LLP
(86) International application number: PCT/JP2019/051301
(87) International publication number: WO 2020/138366

(57) **Abstract**

The present invention provides a high broussonetine-content stevia plant comprising broussonetine at higher content as compared with the wild type stevia species. The present invention also provides a method of producing such a high broussonetine-content stevia plant, and dried leaf and an extract obtainable from such a plant.

## Description

### TECHNICAL FIELD

The present invention relates to a stevia plant with high content of broussonetine.

### BACKGROUND ART

Broussonetine is a pyrrolidine alkaloid contained in the barks or the like of *Broussonetia kazinoki* Sieb. (Non-Patent Literatures 1 to 3), and is expected to be effective for tumor, diabetes mellitus, viral disease, etc. because of having glycosidase inhibitory activity. However, it has not been reported that other plants contain broussonetine.

### CITATION LIST

### Non-Patent Literature

Non-Patent Literature 1: Shibano et al., Chem Pharm Bull. 1997;45(4):700-5
Non-Patent Literature 2: Shibano et al., Chem Pharm Bull. 1998;46(6):1048-50
Non-Patent Literature 3: Tsukamoto et al., Chem Pharm Bull. 2001;49(4):492-6

### DISCLOSURE OF THE INVENTION

### Problems To Be Solved By The Invention

In response to recently growing health consciousness, there have been increasing needs for highly functional foods.

### Means for Solving the Problems

The present invention provides a high broussonetine-content stevia plant containing broussonetine at high content, a method of producing the plant, and a method of screening for the plant.

In one aspect, the present invention provides the following.
[1] A variant stevia plant having a higher broussonetine content than that of the wild type.
[2] The stevia plant according to [1],
   comprising 1% or more rebaudioside D per unit mass of dried leaf, and/or
   comprising more than 0.6% rebaudioside M per unit mass of dried leaf.
[3] The plant according to [1] or [2], wherein the broussonetine is broussonetine F, broussonetine H, broussonetine R, broussonetine T or a combination thereof.
[4] The plant according to any one of [1] to [3], wherein the plant is a non-genetically modified plant.
[5] The plant according to any one of [1] to [4], wherein the plant includes a stevia plant subjected to a mutagenesis treatment and a progeny plant thereof.
[6] A seed, tissue, tissue culture or cell of a plant according to any one of [1] to [5].
[7] The tissue, tissue culture or cell according to [6], which is selected from an embryo, a meristem cell, pollen, leaf, root, root apex, petal, protoplast, leaf section and callus.
[8] A method of producing a stevia plant having a higher broussonetine-content than that of the wild type, the method comprising a step of crossing a plant according to any one of [1] to [5] with a second stevia plant.
[9] The method according to [7], wherein the second plant is a plant according to any one of [1] to [5].
[10] An extract of a plant according to any one of [1] to [5], or of a seed, tissue, tissue culture or cell according to [6] or [7].
[11] A method of producing a broussonetine-containing extract, comprising a step of obtaining an extract from a stevia plant or from seed, tissue, tissue culture or cell thereof.
[12] The method according to [11], wherein the stevia plant is a plant according to any one of [1] to [5].
[13] The method according to [11] or [12], wherein the broussonetine-containing extract further contains steviol glycosides.
[14] A method of producing broussonetine, comprising a step of purifying broussonetine from a broussonetine-containing extract obtained by a method according to any one of [11] to [13].
[15] A method of producing a food or beverage, a sweetener composition, a flavor or a medicament containing broussonetine, comprising:
   a step of providing a broussonetine-containing extract by use of a method according to any one of [11] to [13];
   a step of optionally purifying the broussonetine-containing extract to provide broussonetine; and
   a step of adding the extract or broussonetine to a raw material for the food or beverage, sweetener composition, flavor or medicament.
[16] A method of screening for a high rebaudioside D-content stevia plant and/or a high rebaudioside M-content stevia plant, comprising a step of determining the content of broussonetine in a test stevia plant.
[17] The method according to [16], wherein the step of determining the content of broussonetine is performed by the use of chromatography or mass spectrometry.
[18] The method according to [16] or [17], further comprising a step of determining the content of rebaudioside D and/or rebaudioside M in a tissue of the test stevia plant.

### ADVANTAGEOUS EFFECTS OF THE INVENTION

The present invention has revealed that stevia plants contain broussonetine, and thereby enables the diversification of a supply source for broussonetine. Since stevia plants with high broussonetine content tend to also have a high content of rebaudioside D and rebaudioside M, the present invention enables the obtainment of, screening for, etc. a stevia plant with high content of such beneficial components.

### DESCRIPTION OF EMBODIMENTS

Hereinafter, the present invention will be described in detail. The embodiments are given below merely for illustrating the present invention and are not intended to limit the present invention by such embodiments. The present invention can be carried out in various modes without departing from the spirit of the present invention.

Note that all documents, as well as laid-open application publications, patent application publications, and other patent documents cited herein shall be incorporated herein by reference. The present specification incorporates the contents of the specification and the drawings of Japanese Patent Application No. 2018-248697, filed on December 28, 2018, from which the present application claims priority.

### 1. High broussonetine-content stevia plant

In one aspect, the present invention provides a variant stevia plant having a higher broussonetine content than that of the wild type (hereinafter, referred to as the "plant of the present invention" or "stevia plant of the present invention").

Examples of the broussonetine include broussonetine A, B, C, D, E, F, G, H, I, J, K, L, M, N, O, P, Q, R, S, T, U, and V. The broussonetine is preferably broussonetine F, H, R and/or T. The broussonetine is more preferably broussonetine F, H and/or T.

Broussonetine can be extracted and quantified by various known approaches, for example, approaches described in Non-Patent Literatures 1 to 3, or an approach described below in the Examples.

Broussonetine can be extracted and quantified by the following approach without limitations (hereinafter, referred to as "measurement method A"): first, fresh leaves of the stevia plant are dried by freeze drying, and homogenized dry matter thereof is added into methanol having a final concentration of 75%. After homogenization in a bead homogenizer and centrifugation at 15,000 rpm for 10 minutes, the supernatant is recovered. The supernatant is applied to already conditioned MonoSpin® C18 column (GL Sciences Inc.) and centrifuged at 5000 g for 2 minutes. Then, the eluate is recovered, filtered through a 0.2 µm filter, and subjected to LC-MS analysis. LC-MS analysis is conducted under the following conditions.

**Table 1 LC analysis conditions**

| Analysis apparatus | Ultimate 3000 |
|---|---|
| Column | InertSustain® AQ-C18 (2.1 × 150 mm, particle size: 3 µm, GL Sciences Inc.) |
| Column temperature | 40°C |
| Eluant | Mobile phase A: 0.1% aqueous formic acid solution |
| | Mobile phase B: acetonitrile |
| Mobile phase flow rate | 0.2 mL/min |
| Sample injection volume | 2 µL |

**Table 2 LC gradient program**

| Time (min) | Mobile phase A (%) | Mobile phase B (%) |
|---|---|---|
| 0.0 | 98 | 2 |
| 3.0 | 98 | 2 |
| 30.0 | 2 | 98 |
| 35.0 | 2 | 98 |
| 35.1 | 98 | 2 |
| 40.0 | 98 | 2 |

**Table 3 MS analysis conditions**

| Analysis apparatus | Q Exactive |
|---|---|
| Measurement time | 3 to 30minutes |
| Ionization method | ESI |
| Full scan resolution | 70000 |
| MS/MS scan resolution | 17500 |
| MS/MS precursor selection | Data Dependent Scan (Top 10) |
| Dynamic Exclusion | 20 seconds |

The content of broussonetine contained in the plant of the present invention is not particularly limited as long as the content is higher than that of the wild type. For example, the peak intensity in measurement by the measurement method A may be 50000 or more, 60000 or more, 300000 or more, 1500000 or more, or the like for broussonetine F, 7000 or more, 8000 or more, 40000 or more, 400000 or more, or the like for broussonetine H, and 40000 or more, 50000 or more, 300000 or more, 1500000 or more, or the like for broussonetine T. The upper limit of the content of broussonetine is not particularly limited, and for example, the peak intensity in measurement by the measurement method A may be 200000000 or the like for broussonetine F, 30000000 or the like for broussonetine H, and 30000000 or the like for broussonetine T.

For example, the ratio of peak intensity in measurement by the measurement method A may be 0.01% or more, 0.02% or more, 0.03% or more, 0.05% or more, 0.08% or more, 0.1% or more, 0.2% or more, 0.3% or more, 0.4% or more, 0.5% or more, 0.6% or more, 0.7% or more, 0.8% or more, 0.9% or more, 1.0% or more, 2.0% or more, 3.0% or more, 4.0% or more, or the like for broussonetine F, 0.001% or more, 0.002% or more, 0.01% or more, 0.04% or more, 0.05% or more, 0.06% or more, 0.07% or more, 0.08% or more, 0.09% or more, 0.1% or more, 0.2% or more, 0.3% or more, or the like for broussonetine H, and 0.005% or more, 0.01% or more, 0.02% or more, 0.03% or more, 0.04% or more, 0.05% or more, 0.06% or more, 0.07% or more, 0.08% or more, 0.09% or more, 0.1% or more, 0.2% or more, 0.3% or more, 0.4% or more, 0.5% or more, 0.6% or more, 0.7% or more, or the like for broussonetine T. The upper limit of the content of broussonetine is not particularly limited, and for example, the peak intensity in measurement by the measurement method A may be 50% or the like for broussonetine F, 7% or the like for broussonetine H, and 8% or the like for broussonetine T.

For a measurement value of broussonetine, it is preferred to use an average value of a plurality of, for example, 2, 3, 4, 5 or more measurement values.

The plant of the present invention may further have at least one of the following features (a) and (b).
(a) Comprising 1% or more, preferably 2% or more, more preferably 3% or more rebaudioside (hereinafter, referred to as "Reb") D per unit mass of dried leaf.
(b) Comprising more than 0.6%, preferably more than 0.7%, more preferably more than 0.8% RebM per unit mass of dried leaf.

In certain embodiments, the plant of the present invention may further have at least one of the following features (1) to (4).
(1) Comprising 3.3% or more of RebD per unit mass of dried leaf (hereinafter, referred to as "plant A of the present invention" or "stevia plant A of the present invention").
(2) Comprising 2.6% or more of RebD and 0.4% or more of RebM per unit mass of dried leaf (hereinafter, referred to as "plant B of the present invention" or "stevia plant B of the present invention").
(3) Comprising 3.7% or more in total of RebD and RebM per unit mass of dried leaf (hereinafter, referred to as "plant C of the present invention" or "stevia plant C of the present invention").
(4) The total mass ratio of RebD and RebM to total steviol glycoside is 37.8% or more (hereinafter, referred to as "plant D of the present invention" or "stevia plant D of the present invention").

Total steviol glycoside (TSG) is a generic name for measurable steviol glycosides and includes neither unknown steviol glycosides nor steviol glycosides present at a level less than the detection limit. Preferably, the total steviol glycoside is any combination of two or more members selected from the group consisting of RebA, RebB, RebD, RebE, RebF, Rebl, RebJ, RebK, RebM, RebN, RebO, RebQ, RebR, dulcoside A, rubusoside, steviol, steviolmonoside, steviolbioside and stevioside. In certain embodiments the total steviol glycoside may consist of, for example, RebA, RebB, RebM, RebD, RebF, RebM and steviol. In other embodiments the total steviol glycoside may consist of RebA, RebB, RebM, RebD, RebF, RebM, RebN, RebO and steviol.

In the plant A of the present invention, the feature "comprising 3.3% or more of RebD per unit mass of dried leaf" means that, for example, RebD is contained at a ratio of 3.3% by mass or more (e.g. 1.65 mg or more) in dried leaf having a predetermined mass (e.g. 50 mg). In this embodiment, the ratio of RebD per unit mass of dried leaf is not limited and may be, for example, 3.3% or more, 3.4% or more, 3.5% or more, 3.6% or more, 3.7% or more, 3.8% or more, 3.9% or more, 4.0% or more, 4.1% or more, 4.2% or more, 4.3% or more, 4.4% or more, 4.5% or more, 4.6% or more, 4.7% or more, 4.8% or more, 4.9% or more, 5.0% or more, 5.1% or more, 5.2% or more, 5.3% or more, 5.4% or more, 5.5% or more, 5.6% or more, 5.7% or more, 5.8% or more, 5.9% or more, 6.0 % or more, or the like, and is preferably 3.6% or more. The upper limit of the ratio of RebD per unit mass of dried leaf is not particularly limited and may be, for example, 20%, 15% or 10%.

In this context, dried leaf refers to leaf having a water content decreased to 3 to 4% by weight by drying fresh leaves of the stevia plant of the present invention.

In the plant B of the present invention, the feature "comprising 2.6% or more of RebD and 0.4% or more of RebM per unit mass of dried leaf" means that, for example, RebD and RebM are contained at ratios of 2.6% by mass or more (e.g. 1.3 mg or more per 50 mg of dried leaf) and 0.4% by mass or more (e.g. 0.2 mg or more per 50 mg of dried leaf), respectively, in dried leaf having a predetermined mass (e.g. 50 mg). In this embodiment, the ratios of RebD and RebM, when indicated by (ratio of RebD:ratio of RebM), per unit mass of dried leaf is not limited and may be, for example, (2.6% or more : 0.4% or more), (2.8% or more : 0.4% or more), (3% or more : 0.4% or more), (3.2% or more : 0.4% or more), (3.4% or more : 0.4% or more), (3.6% or more : 0.4% or more), (3.8% or more : 0.4% or more), (4% or more : 0.4% or more), (4.2% or more : 0.4% or more), (4.4% or more : 0.4% or more), (4.6% or more : 0.4% or more), (4.8% or more : 0.4% or more), (5% or more : 0.4% or more), (2.6% or more : 0.5% or more), (2.8% or more : 0.5% or more), (3% or more : 0.5% or more), (3.2% or more : 0.5% or more), (3.4% or more : 0.5% or more), (3.6% or more : 0.5% or more), (3.8% or more : 0.5% or more), (4% or more : 0.5% or more), (4.2% or more : 0.5% or more), (4.4% or more : 0.5% or more), (4.6% or more : 0.5% or more), (4.8% or more : 0.5% or more), (5% or more : 0.5% or more), (2.6% or more : 0.6% or more), (2.8% or more : 0.6% or more), (3% or more : 0.6% or more), (3.2% or more : 0.6% or more), (3.4% or more : 0.6% or more), (3.6% or more : 0.6% or more), (3.8% or more : 0.6% or more), (4% or more : 0.6% or more), (4.2% or more : 0.6% or more), (4.4% or more : 0.6% or more), (4.6% or more : 0.6% or more), (4.8% or more : 0.6% or more), (5% or more : 0.6% or more), (2.6% or more : 0.7% or more), (2.8% or more : 0.7% or more), (3% or more : 0.7% or more), (3.2% or more : 0.7% or more), (3.4% or more : 0.7% or more), (3.6% or more : 0.7% or more), (3.8% or more : 0.7% or more), (4% or more : 0.7% or more), (4.2% or more : 0.7% or more), (4.4% or more : 0.7% or more), (4.6% or more : 0.7% or more), (4.8% or more : 0.7% or more), (5% or more : 0.7% or more), (2.6% or more : 0.8% or more), (2.8% or more : 0.8% or more), (3% or more : 0.8% or more), (3.2% or more : 0.8% or more), (3.4% or more : 0.8% or more), (3.6% or more : 0.8% or more), (3.8% or more : 0.8% or more), (4% or more : 0.8% or more), (4.2% or more : 0.8% or more), (4.4% or more : 0.8% or more), (4.6% or more : 0.8% or more), (4.8% or more : 0.8% or more), (5 % or more : 0.8 % or more), or the like, and is preferably (3.6% or more : 0.4% or more). The upper limit of the ratio of RebD per unit mass of dried leaf is not particularly limited and may be, for example, 20%, 15% or 10%. The upper limit of the ratio of RebM per unit mass of dried leaf is not particularly limited and may be, for example, 10%, 5% or 3%.

In the plant C of the present invention, the feature "comprising 3.7% or more in total of RebD and RebM per unit mass of dried leaf" means that, for example, the total mass of RebD and RebM contained in dried leaf having a predetermined mass (e.g. 50 mg) is 3.7 % by mass or more (e.g. 1.85 mg or more). In this embodiment, the total ratio of RebD and RebM per unit mass of dried leaf is not limited and may be, for example, 3.7% or more, 3.8% or more, 3.9% or more, 4.0% or more, 4.1% or more, 4.2% or more, 4.3% or more, 4.4% or more, 4.5% or more, 4.6% or more, 4.7% or more, 4.8% or more, 4.9% or more, 5.0% or more, 5.1% or more, 5.2% or more, 5.3% or more, 5.4% or more, 5.5% or more, 5.6% or more, 5.7% or more, 5.8% or more, 5.9% or more, 6.0% or more, 6.1% or more, 6.2% or more, 6.3% or more, 6.4% or more, 6.5% or more, 6.6% or more, 6.7% or more, 6.8% or more, 6.9% or more, 70 % or more, or the like, and is preferably 4.9 % or more. The upper limit of the total ratio of RebD and RebM per unit mass of dried leaf is not particularly limited and may be, for example, 25 %, 20 % or 15 %.

In the plant D of the present invention, the feature "the total mass ratio of RebD and RebM to total steviol glycoside is 37.8% or more" means that, for example, when the total mass of RebD and RebM contained in leaf (e.g. dried leaf or fresh leaf) is indicated by RebD + RebM/TSG% as the ratio to the total mass of steviol glycosides obtained from the leaf, the lower limit of the value of RebD + RebM/TSG is 37.8% or more. In this embodiment, the value of RebD + RebM/TSG is not limited and may be, for example, 37.8% or more, 37.9% or more, 38.0% or more, 38.1% or more, 38.2% or more, 38.3% or more, 38.4% or more, 38.5% or more, 38.6% or more, 38.7% or more, 38.8% or more, 38.9% or more, 39.0% or more, 39.2% or more, 39.4% or more, 39.6% or more, 39.8% or more, 40.0% or more, 40.2% or more, 40.4% or more, 40.6% or more, 40.8% or more, 41.0% or more, 41.2% or more, 41.4% or more, 41.6% or more, 41.8% or more, 42.0% or more, 42.4% or more, 42.8% or more, 43.2% or more, 43.6% or more, 44.0% or more, 44.4% or more, 44.8% or more, 45.2% or more, 45.6% or more, 46.0 % or more, or the like, and is preferably 38.1% or more. The upper limit of the mass ratio of RebD + RebM to total steviol glycoside is not particularly limited and may be, for example, 85%, 75%, 65% or 55%.

RebD and RebM can be extracted in the state of a liquid extract by reacting fresh leaves or dried leaves of the plant of the present invention with a suitable solvent (an aqueous solvent such as water or an organic solvent such as an alcohol, ether or acetone). For the extraction conditions, etc., see a method described in Ohta et al., J. Appl. Glycosci., Vol. 57, No. 3 (2010) or WO2010/038911, or a method described in Examples mentioned later.

RebD and RebM, can be further purified from the liquid extract thus obtained by use of a method known in the art such as a gradient of ethyl acetate or any of other organic solvents:water, high performance liquid chromatography (HPLC), gas chromatography, time-of-flight mass spectrometry (TOF-MS), or ultra (high) performance liquid chromatography (UPLC).

The contents of RebD and RebM can be measured by a method described in Ohta et al., J. Appl. Glycosci., Vol. 57, No. 3 (2010) or WO2010/038911, or a method described in Examples mentioned later. Specifically, fresh leaves can be sampled from a stevia plant of the present invention, followed by measurement by LC/MS-MS.

A plant of the present invention may include not only the whole plant but a plant organ (e.g. a leaf, a petal, a stem, a root, and a seed), a plant tissue (e.g. epidermis, phloem, soft tissue, xylem, vascular bundle, palisade tissue, and spongy tissue), various forms of plant cells (e.g. suspended cultured cells), a protoplast, a leaf section, a callus, and the like. The leaf may be the dried leaf mentioned above.

The plant of the present invention may also include a tissue culture or a cultured plant cell. This is because the plant can be regenerated by culturing such a tissue culture or a cultured plant cell. Examples of the tissue culture or the cultured plant cell of the plant of the present invention include, but are not limited to, embryos, meristem cells, pollens, leaves, roots, root apices, petals, protoplasts, leaf sections and calluses.

### 2. Method of producing a plant of present invention

In an alternative aspect, the present invention provides a method of producing a high broussonetine-content stevia plant having a higher broussonetine content than that of the wild type, the method comprising a step of crossing the stevia plant of the present invention with a second stevia plant (hereinafter, may be referred to as the "production method of the present invention").

The plant produced by the method has the same phenotype as those of the plant of the present invention.

In the production method of the present invention, "hybridizing" means that the plant of the present invention (first generation (S1)) is crossed with a second plant (S1) to obtain a progeny plant thereof (plant produced by the production method of the present invention (second generation (S2)). The hybridizing method is preferably backcross. The "backcross" is an approach of further crossing a progeny plant (S2) generated between the plant of the present invention and the second plant, with the plant of the present invention (i.e. a plant having the genetic feature(s) of the present invention) (S1) to produce a plant having the genetic feature(s) of the present invention. When the second plant (S1) for use in the production method of the present invention has the same phenotype and genetic properties as those of the plant of the present invention, the crossing is substantially backcross. The genetic polymorphism of the present invention is inheritable according to the Mendel's law. In association with this, the phenotype correlating with the genetic polymorphism, i.e. the high broussonetine-content phenotype, is also inheritable according to the Mendel's law.

Alternatively, the plant of the present invention can also be produced by selfing. The selfing can be performed by the self-pollination of the stamen pollen of the plant of the present invention with the pistil of the plant of the present invention.

Since the plant produced by the production method of the present invention has the same phenotype as that of the plant of the present invention, the plant produced by the production method of the present invention can be further crossed with a third stevia plant to produce a high broussonetine-content stevia plant having a higher broussonetine content than that of the wild type. The plant produced by the production method of the present invention may have the same genetic features as those of the plant of the present invention.

In an alternative embodiment, the plant of the present invention may be produced by regenerating a plant by the culture of the tissue culture or the cultured plant cell mentioned above. The culture conditions are the same as those for culturing a tissue culture or a cultured plant cell of the wild type stevia plant and are known in the art (Protocols for In Vitro cultures and secondary metabolite analysis of aromatic and medicinal plants, Method in molecular biology, vo. 1391, pp113-123).

Alternatively, the plant of the present invention may be produced by varying the genome of a wild-type stevia plant by a nongenetic recombination approach. Examples of the "non-genetic modification approach" include a method of inducing a variation in the gene of a host cell (or a host plant) without transfection with a foreign gene. Examples of such a method include a method of allowing a mutagen to act on a plant cell. Examples of such a mutagen include ethylmethanesulfonic acid (EMS) and sodium azide. For example, the ethylmethanesulfonic acid (EMS) can be used at a concentration such as 0.1%, 0.2%, 0.3%, 0.4%, 0.5%, 0.6%, 0.7%, 0.8%, 0.9%, or 1.0% to treat a plant cell. The treatment time is 1 to 48 hours, 2 to 36 hours, 3 to 30 hours, 4 to 28 hours, 5 to 26 hours, or 6 to 24 hours. The procedures themselves of the treatment are known in the art and can be performed by dipping a water-absorbed seed obtained through a water absorption process in a treatment solution containing the mutagen at the concentration described above for the treatment time described above.

An alternative example of the non-genetic modification approach can be a method of irradiating a plant cell with radiation or light beam such as X ray, γ ray, or ultraviolet ray. In this case, a cell irradiated using an appropriate dose (ultraviolet lamp intensity, distance, and time) of ultraviolet ray is cultured in a selective medium or the like, and then, a cell, a callus, or a plant having the trait of interest can be selected. In this operation, the irradiation intensity is 0.01 to 100 Gr, 0.03 to 75 Gr, 0.05 to 50 Gr, 0.07 to 25 Gr, 0.09 to 20 Gr, 0.1 to 15 Gr, 0.1 to 10 Gr, 0.5 to 10 Gr, or 1 to 10 Gr. The irradiation distance is 1 cm to 200 m, 5 cm to 100 m, 7 cm to 75 m, 9 cm to 50 m, 10 cm to 30 m, 10 cm to 20 m, or 10 cm to 10 m. The irradiation time is 1 minute to 2 years, 2 minutes to 1 year, 3 minutes to 0.5 years, 4 minutes to 1 month, 5 minutes to 2 weeks, or 10 minutes to 1 week. The irradiation intensity, distance and time differ depending on the type of radiation or the state of the subject to be irradiated (cell, callus, or plant) and can be appropriately adjusted by those skilled in the art.

Approaches such as cell fusion, anther culture (haploid induction), and remote crossing (haploid induction) are also known in the art.

In general, plant cells may involve a mutation during culture. Therefore, it is preferred to regenerate a plant individual, for more stably maintaining the trait.

The scope of the present invention does not exclude a plant obtained by the ex-post facto genetic recombination (e.g. genome editing) with the plant of the present invention as a host (e.g. a plant further provided with another trait by genetic recombination with the plant of the present invention as a host).

### 3. Method of screening for high broussonetine-content stevia plant or high RebD-content and/or high RebM-content stevia plant

Since the broussonetine content of a stevia plant has correlation with RebD and RebM contents, the high broussonetine-content stevia plant can be screened for on the basis of a RebD or RebM content, or the high RebD-content and/or high RebM-content stevia plant can be screened for on the basis of a broussonetine content. In this context, "screening" means that the plant of the present invention is discriminated from the other plants to select the plant of the present invention.

Thus, in an alternative aspect, the present invention relates to
(A) a method of screening for a high RebD-content stevia plant and/or a high RebM-content stevia plant, comprising a step of determining the content of broussonetine in a test stevia plant (hereinafter, may be referred to as the "screening method A of the present invention"), and
(B) a method of screening for a high broussonetine-content stevia plant, comprising a step of determining the content of RebD and/or RebM in a test stevia plant (hereinafter, may be referred to as the "screening method B of the present invention").

In the screening method A of the present invention, if the broussonetine content is equal to or more than a reference value, it can be determined that the test stevia plant is a high RebD-content and/or high RebM-content plant, and if the broussonetine content is less than the reference value, it can be determined that the test stevia plant is a low RebD-content and/or low RebM-content plant.

The reference value is not particularly limited as long as the high RebD-content and/or high RebM-content stevia plant can be discriminated from the low RebD-content and/or low RebM-content stevia plant. For example, the peak intensity in measurement by the measurement method A may be 500000, 700000, 1000000, 1500000, or the like for broussonetine F, 90000, 120000, 130000, 400000, or the like for broussonetine H, and 400000, 500000, 1000000, 1500000, or the like for broussonetine T.

The reference value, for example, as the ratio of peak intensity in measurement by the measurement method A may be 0.15%, 0.3%, 0.5%, 0.7%, or the like for broussonetine F, 0.02%, 0.025%, 0.03%, 0.04%, or the like for broussonetine H, and 0.1%, 0.2%, 0.3%, 0.4%, or the like for broussonetine T.

For a measurement value of broussonetine, it is preferred to use an average value of a plurality of, for example, 2, 3, 4, 5 or more measurement values.

In the screening method B of the present invention, if the RebD and/or RebM content is equal to or more than a reference value, it can be determined that the test stevia plant is a high broussonetine-content plant, and if the RebD and/or RebM content is less than the reference value, it can be determined that the test stevia plant is a low broussonetine-content plant.

The reference value is not particularly limited as long as the high broussonetine-content stevia plant can be discriminated from the low broussonetine-content stevia plant. For example, the content in dried leaf may be 1% by mass, 2% by mass, 3% by mass, 3.3% by mass, 3.6% by mass, or the like for RebD, and 0.4% by mass, 0.6% by mass, 0.7% by mass, 0.8% by mass, or the like for RebM.

The screening method A of the present invention may further comprise a step of determining the RebD and/or RebM content of a tissue (e.g. a leave) of the test stevia plant tissue for which broussonetine at a level equal to or more than the reference value has been detected. The determination of the RebD and RebM content is as described in the section relating to the plant of the present invention. In this embodiment, the screening method of the present invention may be applied to daughter plants obtained by selecting individuals with a higher content of RebD and/or RebM from among the test stevia plants in which broussonetine at a level equal to or more than the reference value is detected, and crossing the selected individuals with another stevia plants. Thus, the screening method of the present invention may comprise one or more of the following steps.
(i) Determining the broussonetine content in a test stevia plant;
(ii) determining the RebD and/or RebM content of the test stevia plant tissue in which broussonetine at a level equal to or more than the reference value has been detected;
(iii) selecting an individual with a higher content of RebD and/or RebM from among the test stevia plants in which broussonetine at a level equal to or more than the reference value has been detected;
(iv) crossing the selected individual with a higher content of RebD and/or RebM with another stevia plant;
(v) determining the broussonetine content in daughter plants obtained by crossing,
(vi) determining the RebD and/or RebM content of the tissue of the daughter plants in which broussonetine at a level equal to or more than the reference value has been detected,
(vii) selecting individuals having a higher RebD and/or RebM content from among the daughter plants in which broussonetine at a level equal to or more than the reference value is detected.

Individuals with a high content of RebD and/or RebM of choice may be, for example, up to 50%, up to 40%, up to 30%, up to 20%, up to 10%, up to 5%, up to 4%, up to 3%, up to 2%, or up to 1% of the test stevia plants in which broussonetine at a level equal to or more than the reference value has been detected, with respect to the high content of RebD and/or RebM. Other stevia plants to be crossed may or may not contain broussonetine at a level equal to or more than the reference value. In the above embodiment, steps (iv) to (vii) can be repeated a plurality of times. In this way, stevia plants with a higher content of RebD and/or RebM can be screened.

The screening method B of the present invention may further comprise a step of determining the broussonetine content of a tissue (e.g. a leave) of the test stevia plant tissue for which RebD and/or RebM at a level equal to or more than the reference value has been detected. The determination of the broussonetine content is as described in the section relating to the plant of the present invention. In this embodiment, the screening method of the present invention may be applied to daughter plants obtained by selecting individuals with a higher content of broussonetine from among the test stevia plants in which RebD and/or RebM at a level equal to or more than the reference value is detected, and crossing the selected individuals with another stevia plants. Thus, the screening method of the present invention may comprise one or more of the following steps.
(i) Determining the RebD and/or RebM content in a test stevia plant;
(ii) determining the broussonetine content of the test stevia plant tissue in which RebD and/or RebM at a level equal to or more than the reference value has been detected;
(iii) selecting an individual with a higher content of broussonetine from among the test stevia plants in which RebD and/or RebM at a level equal to or more than the reference value has been detected;
(iv) crossing the selected individual with a higher content of broussonetine with another stevia plant;
(v) determining the RebD and/or RebM content in daughter plants obtained by crossing,
(vi) determining the broussonetine content of the tissue of the daughter plants in which RebD and/or RebM at a level equal to or more than the reference value has been detected,
(vii) selecting individuals having a higher broussonetine content from among the daughter plants in which RebD and/or RebM at a level equal to or more than the reference value is detected.

Individuals with a high content of broussonetine of choice may be, for example, up to 50%, up to 40%, up to 30%, up to 20%, up to 10%, up to 5%, up to 4%, up to 3%, up to 2%, or up to 1% of the test stevia plants in which RebD and/or RebM at a level equal to or more than the reference value has been detected, with respect to the high content of broussonetine. Other stevia plants to be crossed may or may not contain RebD and/or RebM at a level equal to or more than the reference value. In the above embodiment, steps (iv) to (vii) can be repeated a plurality of times. In this way, stevia plants with a higher content of broussonetine can be screened.

In the screening method of the present invention, the test stevia plant may be a natural plant or a non-transgenic plant. Non-transgenic plants are as described in the section relating to the plant of the present invention.

In the screening method of the present invention, the test stevia plant may include a stevia plant subjected to a mutagenesis treatment and a progeny plant thereof. The mutagenesis treatment is as described in the section relating to the plant of the present invention, and includes treatment with a mutagen, treatment with radiation or irradiation with light, and the like.

### 4. Method of producing a plant-derived extract, and product comprising the extract

In a further aspect, the present invention provides a method of producing a broussonetine-containing extract, comprising a step of obtaining an extract from the plant of the present invention, or a seed or a leaf (e.g. dried leaf or fresh leaf) of the plant (hereinafter, may be referred to as the "extract production method of the present invention"). The present invention further provides a method of producing broussonetine, comprising a step of purifying broussonetine from an extract obtained by the extract production method of the present invention (hereinafter, may be referred to as the "broussonetine production method of the present invention").

Specifically, the present invention provides a method of producing broussonetine, comprising a step of obtaining an extract containing broussonetine from the high broussonetine-content stevia plant of the present invention, the high broussonetine-content stevia plant screened for by the screening method of the present invention, or the high broussonetine-content stevia plant produced by the method of the present invention.

The extract containing broussonetine can be obtained by reacting a fresh leaf or dried leaf of the plant of the present invention with a suitable solvent (an aqueous solvent such as water or an organic solvent such as an alcohol, ether or acetone). For the extraction conditions, etc., see a method described in Non-Patent Literatures 1 to 3, or a method described in the Examples mentioned later.

The broussonetine can be purified from the extract containing broussonetine by use of a method known in the art such as a gradient of ethyl acetate or any of other organic solvents:water, high performance liquid chromatography (HPLC), gas chromatography, time-of-flight mass spectrometry (TOF-MS), or ultra (high) performance liquid chromatography (UPLC).

The extract obtained by the extract production method of the present invention (hereinafter, may be referred to as the "extract of the present invention") comprises broussonetine at higher content as compared with the wild type stevia species.

The extract of the present invention may comprise broussonetine at higher content by 150% or more, 200% or more, 300% or more, 400% or more, 500% or more, 600% or more, 700% or more, 800% or more, 900% or more, 1100% or more, 1200% or more, 1300% or more, 1400% or more, 1500% or more, 1600% or more, 1700% or more, 1800% or more, 1900% or more, 2000% or more, 2100% or more, 2200% or more, 2300% or more, 2400% or more, 2500% or more, 2600% or more, 2700% or more, 2800% or more, 2900% or more, 3000% or more, 3100% or more, 3200% or more, 3300% or more, 3400% or more, 3500% or more, 3600% or more, 3700% or more, 3800% or more, 3900% or more, 4000% or more, 4100% or more, 4200% or more, 4300% or more, 4400% or more, 4500% or more, 4600% or more, 4700% or more, 4800% or more, 4900% or more, or 5000 % or more as compared with an extract obtained from the wild type stevia species. The extract of the present invention and the extract obtained from the wild type stevia species may be those obtained by the same process.

The extract of the present invention may further contain steviol glycosides. The steviol glycosides are compounds containing a steviol backbone conjugated with sugar, and nonlimiting examples thereof include RebA, RebB, RebD, RebE, RebF, Rebl, RebJ, RebK, RebM, RebN, RebO, RebQ, RebR, dulcoside A, rubusoside, steviol, steviolmonoside, steviolbioside, and stevioside. Preferred examples of the steviol glycosides contained in the extract of the present invention include RebD and RebM.

The extract of the present invention thus obtained and/or broussonetine obtained by the method of producing broussonetine according to the present invention can be mixed with other component(s) to produce a novel medicament, flavor or food or beverage, etc. with increased content of broussonetine. Accordingly, in an alternative aspect, the present invention provides a method of producing a medicament, a flavor or a food or beverage, comprising a step of mixing the extract of the present invention and/or broussonetine obtained by the method of producing broussonetine according to the present invention with other component(s). The present invention further provides a novel medicament, flavor or food or beverage with increased content of broussonetine, obtained by the production method. In this context, the food or beverage means a drink and a food. Thus, in a certain embodiment, the present invention provides a novel medicament, flavor, drink or food and also provides a method of producing the medicament, the flavor, the drink or the food. In one embodiment, the medicament, the flavor or the food or beverage of the present invention containing broussonetine has glycosidase inhibitory activity and is useful in the treatment of tumor, diabetes mellitus, viral disease, etc.

### EXAMPLES

Hereinafter, the present invention will be described with reference to Experimental Examples, Examples, etc. However, the present invention is not limited by these specific embodiments.

### Example 1 - Measurement of broussonetine content

The broussonetine contents of dried leaves of stevia plants of various lines were measured. Specifically, an appropriate amount of fresh leaves was sampled from each individual, 0.25 g of fresh leaves was dried by freeze drying, and 0.05 g of homogenized dry matter thereof was added into methanol having a final concentration of 75%. After homogenization in a bead homogenizer and centrifugation at 15,000 rpm for 10 minutes, the supernatant was recovered. The supernatant was applied to already conditioned MonoSpin® C18 column (GL Sciences Inc.) and centrifuged at 5000 g for 2 minutes. Then, the eluate is recovered and filtered through a 0.2 µm filter. The resultant was used as a sample for LC-MS analysis. In the LC-MS analysis, Q Exactive (Thermo Fisher Scientific Inc.) connected with Ultimate 3000 RSLC (Thermo Fisher Scientific Inc.) was used. The analysis conditions and the analysis results are shown below. The analysis results are indicated by an average peak intensity value in mass chromatograms obtained by 5 repetitive experiments and the ratio of peak intensity (%) of each broussonetine to the total peak intensity. All of the stevia plants used are progeny of individuals genetically modified by mutagenesis treatment with ethylmethanesulfonic acid (EMS).

**Table 4 LC analysis conditions**

| Analysis apparatus | Ultimate 3000 |
|---|---|
| Column | InertSustain® AQ-C18 (2.1 × 150 mm, particle size: 3 µm, GL Sciences Inc.) |
| Column temperature | 40°C |
| Eluant | Mobile phase A: 0.1% aqueous formic acid solution |
| | Mobile phase B: acetonitrile |
| Mobile phase flow rate | 0.2 mL/min |
| Sample injection volume | 2 µL |

**Table 5 LC gradient program**

| Time (min) | Mobile phase A (%) | Mobile phase B (%) |
|---|---|---|
| 0.0 | 98 | 2 |
| 3.0 | 98 | 2 |
| 30.0 | 2 | 98 |
| 35.0 | 2 | 98 |
| 35.1 | 98 | 2 |
| 40.0 | 98 | 2 |

**Table 6 MS analysis conditions**

| Analysis apparatus | Q Exactive |
|---|---|
| Measurement time | 3 to 30minutes |
| Ionization method | ESI |
| Full scan resolution | 70000 |
| MS/MS scan resolution | 17500 |
| MS/MS precursor selection | Data Dependent Scan (Top 10) |
| Dynamic Exclusion | 20 seconds |

**Table 7 Analysis results (Peak intensity)**

| Individual number | Broussonetine F (accurate mass: 361.2) | Broussonetine H (accurate mass: 359.2) | Broussonetine T (accurate mass: 377.2) | Total peak intensity |
|---|---|---|---|---|
| 1 | 735689 | 126619 | 1010752 | 519351969 |
| 2 | 15871982 | 2318909 | 2689642 | 392703166 |
| 3 | 7319853 | 141755 | 2175946 | 396369199 |
| 4 | 586949 | 46292 | 441933 | 247775106 |
| 5 | 402432 | 92750 | 341417 | 571965250 |
| 6 | 313953 | 116100 | 324744 | 672398188 |

**Table 8 Analysis results (Ratio of peak intensity (%))**

| Individual number | Broussonetine F | Broussonetine H | Broussonetine T |
|---|---|---|---|
| 1 | 0.138 | 0.022 | 0.201 |
| 2 | 4.099 | 0.595 | 0.685 |
| 3 | 1.895 | 0.035 | 0.554 |
| 4 | 0.269 | 0.021 | 0.194 |
| 5 | 0.077 | 0.017 | 0.060 |
| 6 | 0.036 | 0.014 | 0.032 |

As shown in the results, the stevia plants were found to contain each type of broussonetine.

### Example 2 - Measurement of steviol glycoside content

An appropriate amount of fresh leaves was sampled from each of the same individuals as those of Example 1, 0.25 g of fresh leaves was dried by freeze drying, and 0.05 g of homogenized dry matter thereof was added into pure water. Extraction by ultrasonic treatment for 20 minutes, and centrifugation and filtration were performed to obtain 0.33 mL of a liquid extract. The concentrations of RebA, RebB, RebC, RebD, RebF, RebM, RebN and RebO were quantitatively determined by LC/MS-MS analysis on this liquid extract in LCMS8050 (Shimadzu Corp.), and the total sum thereof was regarded as the concentration of total steviol glycoside (TSG). The results are shown in the table below. Each numerical value represents % by mass in dried leaf or a ratio (%) to TSG.

**Table 9 Steviol glycoside content**

| Individual number | RebA | RebD | Reb M | TSG | RebD+ RebM | RebD/ TSG | RebM/ TSG | (RebD+RebM)/ TSG |
|---|---|---|---|---|---|---|---|---|
| 1 | 3.49 | 3.85 | 0.63 | 11.52 | 4.48 | 33.46 | 5.45 | 38.91 |
| 2 | 5.42 | 4.13 | 1.13 | 12.45 | 5.26 | 33.21 | 9.05 | 42.26 |
| 3 | 5.72 | 3.10 | 0.92 | 11.71 | 4.02 | 26.46 | 7.86 | 34.32 |
| 4 | 9.00 | 0.59 | 0.60 | 12.30 | 1.20 | 4.84 | 4.90 | 9.73 |
| 5 | 6.53 | 0.79 | 0.10 | 15.70 | 0.89 | 5.06 | 0.63 | 5.70 |
| 6 | 16.93 | 0.95 | 0.10 | 35.97 | 1.05 | 2.64 | 0.27 | 2.92 |

### Example 3 - Study on correlation of broussonetine content with steviol glycoside content

The presence or absence of the correlation between broussonetine content and steviol glycoside content was studied on the basis of the results obtained in Examples 1 and 2. The correlation coefficient between the content of each broussonetine and the content of each steviol glycoside or TSG is shown in the table below.

**Table 10 Correlation coefficient between broussonetine content (peak intensity) and steviol glycoside content**

| | RebA | RebD | RebM | TSG |
|---|---|---|---|---|
| Broussonetine F | -0.358 | 0.688 | 0.820 | -0.337 |
| Broussonetine H | -0.253 | 0.590 | 0.645 | -0.208 |
| Broussonetine T | -0.510 | 0.829 | 0.907 | -0.464 |

**Table 11 Correlation coefficient between broussonetine content (ratio of peak intensity) and steviol glycoside content**

| | RebA | RebD | RebM | TSG |
|---|---|---|---|---|
| Broussonetine F | -0.357 | 0.674 | 0.829 | -0.350 |
| Broussonetine H | -0.265 | 0.583 | 0.660 | -0.231 |
| Broussonetine T | -0.509 | 0.752 | 0.946 | -0.530 |

As shown in the results, positive correlation was found between the broussonetine content and the RebD and RebM contents.

### INDUSTRIAL APPLICABILITY

The present invention diversifies supply sources for broussonetine and facilitates provision of highly functional medicaments, flavors foods or beverages, etc. containing broussonetine.

## Claims

1. A variant stevia plant having a higher broussonetine content than that of the wild type.

2. The stevia plant according to claim 1,
comprising 1% or more rebaudioside D per unit mass of dried leaf, and/or
comprising more than 0.6% rebaudioside M per unit mass of dried leaf.

3. The plant according to claim 1 or 2, wherein the broussonetine is broussonetine F, broussonetine H, broussonetine R, broussonetine T or a combination thereof.

4. The plant according to any one of claims 1 to 3, wherein the plant is a non-genetically modified plant.

5. The plant according to any one of claims 1 to 4, wherein the plant is a stevia plant subjected to a mutagenesis treatment or a progeny plant thereof.

6. Seed, tissue, tissue culture or cell of a plant according to any one of claims 1 to 5.

7. The tissue, tissue culture or cell according to claim 6, which is selected from an embryo, meristem cell, pollen, leaf, root, root apex, petal, protoplast, leaf section and callus.

8. A method of producing a stevia plant having a higher broussonetine-content than that of the wild type, the method comprising a step of crossing a plant according to any one of claims 1 to 5 with a second stevia plant.

9. The method according to claim 7, wherein the second plant is a plant according to any one of claims 1 to 5.

10. An extract of a plant according to any one of claims 1 to 5, or of a seed, tissue, tissue culture or cell according to claim 6 or 7.

11. A method of producing a broussonetine-containing extract, comprising a step of obtaining an extract from a stevia plant or a seed, a tissue, tissue culture or cell thereof.

12. The method according to claim 11, wherein the stevia plant is a plant according to any one of claims 1 to 5.

13. The method according to claim 11 or 12, wherein the broussonetine-containing extract further contains steviol glycosides.

14. A method of producing broussonetine, comprising a step of purifying broussonetine from a broussonetine-containing extract obtained by a method according to any one of claims 11 to 13.

15. A method of producing a food or beverage, a sweetener composition, a flavor or a medicament containing broussonetine, comprising:
a step of providing a broussonetine-containing extract by use of a method according to any one of claims 11 to 13;
a step of optionally purifying the broussonetine-containing extract to provide broussonetine; and
a step of adding the extract or broussonetine to a raw material for the food or beverage, sweetener composition, flavor or medicament.

16. A method of screening for a high rebaudioside D-content stevia plant and/or a high rebaudioside M-content stevia plant, comprising a step of determining the content of broussonetine in a test stevia plant.

17. The method according to claim 16, wherein the step of determining the content of broussonetine is performed by use of chromatography or mass spectrometry.

18. The method according to claim 16 or 17, further comprising a step of determining the content of rebaudioside D and/or rebaudioside M in a tissue of the test stevia plant.
